Europäisches Patentamt

(19) European Patent Office (11) Numéro de publication : **0 004 223**

Office européen des brevets **B1**

(12) FASCICULE DE BREVET EUROPÉEN

(45) Date de publication du fascicule du brevet :
09.09.81

(21) Numéro de dépôt : 79400116.4

(22) Date de dépôt : 26.02.79

(51) Int. Cl.³ : **A 61 K 9/50, B 01 J 13/02**

(54) **Procédé de fabrication de capsules lipidiques renfermant un matériau biologiquement actif, produits obtenus par ce procédé ainsi que leur utilisation.**

(30) Priorité : 24.02.78 US 881116

(43) Date de publication de la demande :
19.09.79 (Bulletin 79/19)

(45) Mention de la délivrance du brevet :
09.09.81 Bulletin 81/36

(84) Etats contractants désignés :
**CH IT LU NL SE**

(56) Documents cités :
**DE - A - 2 249 552**
**DE - A - 2 332 640**
**FR - A - 2 298 318**
**LU - A - 74 173**

Chemical Abstracts, Vol. 81, N° 14, 17 octobre 1974, COLUMBUS, OHIO, USA
LEUZINGER : « Encapsulated active substances »

& ZA - A - 73 01850 (BAYER)

* Abrégé n° 82398v *

Chemical Abstracts, Vol. 85, N° 2, 26 juillet 1976, COLUMBUS, OHIO, USA
SUZUKI et al. : « Long-acting drug microparticles »

(73) Titulaire : **PAPAHADJOPOULOS, Demetrios P.**
**3170 Condit Street**
**Lafayette California 74549 (US)**

**Szoka, Francis Charles, Jr.**
**375 Leroy Avenue**
**Buffalo, New York 14214 (US)**

(72) Inventeur : **PAPAHADJOPOULOS, Demetrios P.**
**3170 Condit Street**
**Lafayette California 74549 (US)**
Inventeur : **Szoka, Francis Charles, Jr.**
**375 Leroy Avenue**
**Buffalo, New York 14214 (US)**

(74) Mandataire : **Netter, André**
**40, rue Vignon**
**F-75009 Paris (FR)**

Imprimerie Jouve, 17, rue du Louvre, 75001 Paris, France

# Procédé de fabrication de capsules lipidiques renfermant un matériau biologiquement actif, produits obtenus par ce procédé ainsi que leur utilisation

L'invention est relative à des vésicules ou capsules synthétiques en matériau à base de lipides, à leur procédé de fabrication, à un procédé d'encapsulage de matériaux biologiquement actifs et à l'utilisation de ces vésicules ou capsules.

Plusieurs procédés sont connus pour fabriquer des capsules lipidiques synthétiques (liposomes) renfermant des matériaux biologiquement actifs. Par exemple, Robinson, Trans. Faraday Soc. 56 : 1 260-1 264 (1960) et Papahadjopoulos et al. (Biochim. Biophys. Acta, 135, 639, 1967) ont décrit un procédé de préparation des dispersions de phospholipides à partir d'un système à deux phases éther-lipides-eau qui met en jeu l'évaporation de l'éther en faisant barboter de l'azote dans le mélange. On n'a pas essayé d'utiliser ce procédé pour piéger ou capter des matériaux organiques et l'efficacité du point de vue du captage n'a pas été étudiée en détail. Une technique d'évaporation similaire à partir d'un système chloroforme-aqueux à deux phases a été décrite par Chowhan et al., dans Biochim. Biophys. Acta, 266 : 320-342 (1972). Ce procédé met également en jeu l'utilisation d'une phase aqueuse en excès et l'extraction lente de la phase de chloroforme afin de produire une population uniforme de vésicules ou capsules phospholipidiques. Aucun essai n'a été fait pour renfermer une quantité maximale de matériau aqueux et aucune étude n'a été faite concernant le rendement en matériau enfermé obtenu par ce procédé.

Bangham et al. dans J. Mol. Biol., 13 : 238-252 (1965) ont décrit des capsules à base de lipides multilamellaires qui pourraient être caractérisées comme ayant un faible volume et un faible rendement d'encapsulage (10 %) et un espace aqueux restreint (0,0015 à 0,0035 $\mu$m).

Les petites capsules ou vésicules unilamellaires fabriquées par ultrasons décrites initialement par D. Papahadjopoulos et N. Miller (Biochim. Biophys. Acta, 135 : 624-638 (1967) et par de nombreux autres auteurs depuis, présentent un très faible rendement d'encapsulation et ne sont pas appropriées pour encapsuler de grandes macromolécules en raison de leur petit compartiment aqueux (0,025 $\mu$m).

Des capsules à base de lipides préparées par injection dans une solution aqueuse des lipides contenus dans une phase organique ont été décrites par Batzri et Korn (Biochim. Biophys. Acta, 298 : 1 015 (1973) en utilisant de l'éthanol et par Deamer et Bangham dans Biochim. Biophys. Acta, 443 : 629-634 (1976) en utilisant de l'éther. Par ces procédés, on obtient des capsules unilamellaires ou paucilamellaires mais on ne peut obtenir de grands rendements du point de vue de l'encapsulage. Dans le cas de l'injection d'éthanol, ce faible rendement est dû au grand volume aqueux dans lequel l'éthanol est dispersé et à la petite dimension des capsules obtenues par cette technique. Dans le cas du procédé avec injection d'éther, il est dû à une combinaison du grand volume aqueux dans lequel l'éther est injecté, des petites quantités de lipides utilisées dans le procédé et de la façon suivant laquelle les capsules sont fabriquées. Un procédé de préparation de grandes capsules unilamellaires a été décrit par Papahadjopoulos et al. dans Biochim. Biophys. Acta 394 : 483-491 (1975), procédé qui met en jeu un changement de structure unique induit par du calcium dans la capsule à base de lipides mais cette technique est limitée à un seul phospholipide (phosphatidylsérine) et présente également un rendement d'encapsulage relativement faible en raison du procédé de reconstitution des capsules ou vésicules.

Un autre procédé de préparation de capsules à base de lipides décrit dans le DE-C 2 532 317 met en jeu la centrifugation d'une émulsion lipide-eau-éther dans une phase aqueuse. L'inconvénient de ce procédé est qu'une vitesse élevée de centrifugation est requise et qu'une quantité importante de l'émulsion lipide-eau est retenue à l'interface et ne pénètre pas dans la phase aqueuse. Ceci réduit le pourcentage de matériau encapsulé.

Le US-A 3 804 776 décrit un procédé de préparation d'aminoacides ou de polypeptides encapsulés à l'aide d'huile et de matières grasses en dispersant des poudres du matériau désiré destiné à être encapsulé dans un mélange fondu des matières grasses ou de l'huile et en versant ensuite le mélange fondu dans de l'eau. Le matériau encapsulé est contenu à l'intérieur de gouttelettes de lipides relativement grandes, ce qui limite leur utilisation à l'administration orale à un animal. Le procédé est d'utilisation quelque peu restreinte en ce qu'il est apparemment limité à l'encapsulage de poudres et les lipides ne forment pas une couche double.

Enfin le US-A 4 016 100 décrit l'encapsulage de certains produits pharmaceutiques dans des capsules à base de lipides en congelant la dispersion phospholipidique aqueuse du produit pharmaceutique et des lipides. Les composés pharmaceutiques décrits pour être encapsulés selon ce procédé présentent généralement un coefficient de partage élevé dans une phase organique à partir d'eau. En conséquence, il serait vraisemblable que le matériau destiné à être encapsulé devrait pénétrer dans les couches doubles phospholipidiques des capsules. Théoriquement, ceci devrait fournir un degré élevé d'encapsulage, mais il reste la question de la bio-disponibilité de tout le matériau encapsulé. Il est également vraisemblable que l'on n'obtiendrait pas des taux relativement élevés d'encapsulage si le procédé était appliqué pour encapsuler des produits pharmaceutiques qui sont de nature plus polaire et moins susceptibles de pénétrer les couches doubles de la capsule.

Par le procédé selon l'invention, des capsules à base de lipides oligolamellaires (liposomes synthétiques) peuvent être fabriquées rapidement, facilement, sous des conditions douces, avec des rendements élevés et de telle manière qu'elles incorporent ou enferment un pourcentage élevé d'une grande variété de matériaux biologiquement actifs. Par exemple, par le procédé selon l'invention, on peut encapsuler des composés pharmaceutiquement actifs et des compositions de ces derniers, des hydrates de carbone, des nucléotides, des polynucléotides (naturels et synthétiques), des pesticides, y compris des fongicides, des insecticides, des miticides, des nématocides et des mollusicides, des agents de fertilisation solubles dans l'eau et des agents nutritifs agricoles, des peptides, des protéines, des enzymes, des virus et analogues. De nombreux matériaux parmi ces derniers ne pénètrent pas normalement à travers la membrane de cellules et peuvent être inactivés en circulation à l'intérieur d'un organisme vivant ou par contact avec des cultures de tissus et d'organes. Dans le cas de pesticides et d'engrais ou d'agents de fertilisation pour l'agriculture, ils peuvent être enlevés de l'aire d'application par la pluie ou l'irrigation. L'encapsulage de tels matériaux les protège d'une inactivation ou d'un enlèvement, c'est-à-dire conserve leur bio-disponibilité. Des cellules bactériennes telles que C. parvum et E. coli et analogues peuvent également être encapsulées par le procédé selon l'invention à des fins de protection et de bio-disponibilité.

Le procédé selon l'invention peut également être utilisé pour encapsuler des préparations cosmétiques qui peuvent être utilisées comme décrit dans le Brevet Etats-Unis n° 3 957 971.

Conformément au procédé d'encapsulage de matériaux biologiquement actifs dans des capsules lipidiques oligolamellaires synthétiques selon l'invention :

— on prépare un mélange dans un solvant organique d'un composé formateur de la paroi des capsules et un mélange aqueux du matériau biologiquement actif destiné à être encapsulé, le rapport de la phase organique à la phase aqueuse étant tel qu'il permette l'obtention d'une émulsion du type eau-dans-huile ;

— on forme une émulsion homogène dudit mélange, du type de celle produite par exposition à des ondes ultrasonores ;

— on extrait le solvant organique de l'émulsion, le mélange résultant présentant le caractère d'un gel ; et

— on transforme le mélange analogue à un gel en des capsules oligolamellaires, synthétiques, encapsulant le matériau biologiquement actif.

L'invention porte également sur le matériau intermédiaire analogue à un gel, sur les capsules lipidiques synthétiques obtenues, leur utilisation, ainsi que sur les compositions supports comprenant les capsules synthétiques en tant qu'ingrédient actif.

Par l'expression « matériau biologiquement actif » telle qu'utilisée dans la description, on doit entendre un composé ou une composition qui, quand il ou elle est présent(e) en quantité efficace, réagit avec et/ou agit sur des cellules et des organismes vivants.

Par l'expression « capsules lipidiqes oligolamellaires, synthétiques, (liposomes) », telle qu'utilisée dans la description, on entend des capsules lipidiques fabriquées artificiellement, préparées au laboratoire et caractérisées en partie par une seule couche ou un nombre peu élevé de couches lipidiques bimoléculaires formant les parois des capsules.

Le procédé selon l'invention est utilisé pour fabriquer des capsules lipidiques synthétiques oligolamellaires ou unilamellaires qui, à leur tour, sont utilisées avantageusement dans de nombreux procédés. Par exemple, les capsules lipidiques préparées conformément au procédé selon l'invention peuvent être utilisées pour augmenter la bio-disponibilité des médicaments, pour favoriser le remplacement d'enzymes, l'administration de médicaments oraux, l'administration de médicaments locaux. Les capsules lipidiques obtenues par le procédé selon l'invention peuvent également être utilisées pour encapsuler des préparations cosmétiques, des pesticides, des composés agissant sur la croissance des plantes distribués de façon lente et prolongée, et analogues.

De façon très résumée, le procédé selon l'invention fait appel d'abord à la formation de « micelles inverties » dans une phase organique puis à l'extraction de la phase organique. Le système se transforme alors spontanément en une structure du type couche double ou bicouche, avec une quantité importante de phase aqueuse encapsulée dans des capsules oligolamellaires de grande dimension. L'avantage de ce procédé est qu'il fournit un rendement élevé du point de vue de l'encapsulage de la phase aqueuse et fournit de grandes capsules stables. Les phospholipides sont des molécules excellentes pour la formation des « micelles inverties » et ensuite pour la formation ultérieure de la bicouche des capsules.

Le procédé selon l'invention est mis en œuvre comme suit :

Dans la première étape du procédé de l'invention, on prépare un mélange dans un solvant organique d'une composition de lipides propre à former la paroi des capsules et un mélange aqueux du matériau biologiquement actif et destiné à être encapsulé dans la capsule. Les composés formateurs de parois de capsules sont généralement connus comme le sont leurs procédés de préparation. Par exemple, des phospholipides quelconques ou des composés lipidiques quelconques peuvent être utilisés pour former les parois de capsules. Parmi de tels composés formateurs de parois, on peut citer les suivants : la phosphatidylcholine (appelée ci-après « PC »), se produisant naturellement ou préparée synthétiquement, l'acide phosphatidique (appelé ci-après « PA »), la lysophosphatidylcholine, la phosphaditylsérine (appelée ci-après « PS »), la phosphatidyléthanolamine (appelée ci-après « PE »), les sphingolipides, le phosphaditylglycérol (appelé ci-après « PG »), la spingomyéline, le cardiolipide, les glycolipides, les gangliosides, les cérébrosides et analogues utilisés soit seuls, soit mélangés entre eux, tel que les phospholipides de soja (Asolectine, Associated Concentrates). En outre, d'autres lipides tels que les stéroïdes, le cholestérol, des amines aliphatiques telles que les amines aliphatiques à longue chaîne et des acides carboxyliques, des sulfates et phosphates à longue chaîne, le dicétyl phosphate, l'hydroxytoluène butylé, le tocophérol, le rétinol, et des composés isoprénoïdes peuvent être mélangés avec les composés phospholipidiques pour conférer certaines propriétés désirées et connues aux capsules à fabriquer. En outre, des phospholipides synthétiques contenant soit des portions aliphatiques altérées tels que des groupes hydroxyles, des chaînes carbonées latérales, des dérivés cyclo, des dérivés aromatiques, des éthers, des amides, des dérivés poly-insaturés, des dérivés halogénés, soit des portions hydrophiles altérées contenant des groupes hydrate de carbone, glycol, phosphate, phosphonate, amine quaternaire, sulfate, sulfonate, carboxy, amine, sulfhydryle, imidazole et des combinaisons de tels groupes peuvent être soit substitués, soit mélangés aux phospholipides mentionnés ci-dessus et utilisés dans le procédé selon l'invention. Il est clair de ce qui précède que la composition chimique du constituant lipidique des capsules préparées par le procédé selon l'invention peut varier dans une plage large sans diminution appréciable du pourcentage de matériau encapsulé bien que la dimension de la capsule puisse être affectée par la composition en lipides. Un mélange avantageux utilisé et représentatif des mélanges de lipides avantageusement utilisés dans le procédé selon l'invention, est composé de PS et PC, ou PG et PC comme identifiés ci-dessus (avantageusement dans un rapport molaire 1/4 dans chaque cas). Les PC, PG, PA et PE peuvent être obtenus à partir de jaune d'œuf purifié. Les PC et PG synthétiques saturés, tels que le dipalmitoyle, peuvent également être utilisés. D'autres lipides amphipathiques qui peuvent être utilisés, avantageusement également dans des rapports molaires 1/4 avec du PC, sont les gangliosides, globosides, acides gras, stéarylamines, alcools à longue chaîne et analogues.

La composition formatrice des parois du liposome (ou capsule) peut être fournie initialement dissoute dans tout solvant inerte qui peut être extrait sensiblement en totalité du composé lipidique ou phospholipidique quand désiré. De tels solvants peuvent être choisis parmi une grande variété d'éthers, d'esters, d'alcools, de cétones, d'hydrocarbures (aromatiques et aliphatiques, y compris les hydrocarbures fluorés), et les silicones dans lesquels une phase aqueuse ne présente pas une solubilité appréciable. Les solvants peuvent être utilisés seuls ou en mélange. Pour chaque solvant ou mélange de solvants, cependant, la proportion optimale de lipides, volume aqueux, et solvant est différente et doit être déterminée dans chaque cas expérimentalement ou par tâtonnement, comme les spécialistes le comprendront. Par le terme « solvant inerte » tel qu'utilisé ici, on doit entendre un solvant pour le lipide ou phospholipide, qui n'interfère pas ou n'affecte pas de façon néfaste le déroulement désiré du procédé selon l'invention.

Les phospholipides ou lipides ainsi que tout additif soluble dans les lipides, sont avantageusement séparés de leur solvant par évaporation et déposés sur les parois d'un réacteur approprié. La phase organique, dans laquelle les « capsules produites par évaporation inverse de phase » selon l'invention seront formées, est alors ajoutée dans le réacteur, cette phase étant un solvant organique inerte pour les lipides et phospholipides, comme décrit ci-dessus. Lors de l'opération de mélange, on obtient une dissolution des constituants lipidiques des capsules à fabriquer précédemment déposés sur les parois du réacteur. Un certain nombre de solvants organiques inertes sont utilisés de préférence pour constituer la phase organique, conformément au procédé selon l'invention, en fonction des conditions suivantes mises en œuvre pour le procédé.

Pour des conditions à température basse, c'est-à-dire pour une extraction ultérieure de la phase organique à des températures relativement basses, le diéthyl éther est avantageux bien que le chloroforme ou le tétrahydrofuranne puisse également être utilisé de façon avantageuse. Pour une procédure à plus haute température, on préfère utiliser en tant que solvant organique inerte de l'éther isopropylique, particulièrement pour préparer des capsules lipidiques contenant des phospholipides saturés en tant que constituant lipidique. Après la dissolution du phospholipide ou du lipide pour former la phase organique, une phase aqueuse est ajoutée pour obtenir un mélange hétérogène à deux phases. La phase aqueuse contient en dissolution/suspension les composés ou compositions devant être encapsulés dans la capsule lipidique synthétique fabriquée par le procédé selon l'invention. De préférence, la phase aqueuse est tamponnée à un pH approprié pour maintenir dans un état stable le matériau destiné à être encapsulé. La force ionique de la phase aqueuse a une influence sur le rendement d'encapsulage du procédé selon l'invention. En règle générale, plus la force ionique de la phase aqueuse est élevée, plus le pourcentage d'encapsulage est faible. Par exemple, avec du chlorure de sodium 15 mM, on peut encapsuler environ 60 % de la phase aqueuse tandis qu'avec du chlorure de sodium 500 mM seulement environ 20 % de la phase aqueuse peuvent être encapsulés. Ainsi, pour un encapsulage maximal de macromolécules, un tampon de force ionique faible (inférieure à 0,3) est utilisé de préférence. Le rendement d'encapsulage dépend également quelque peu de la concentration de lipides ou phospholipides présents dans le système à deux phases. De préférence, la proportion du constituant lipidique ou phospholipidique est comprise entre environ 0,5 mg et environ 50 mg/ml de solvant organique inerte. De façon avantageuse, le rapport de la phase organique à la phase aqueuse est compris entre environ 2/l à environ 20/l (volume/volume) et, de préférence, est environ 4/l, pour former une émulsion eau-dans-huile.

Le mélange hétérogène à deux phases obtenu comme décrit ci-dessus est ensuite émulsifié pour obtenir une émulsion du type de celle obtenue par rayonnement par ondes ultrasonores. De préférence, ceci est réalisé en utilisant un dispositif ultrasonore du type à bain (« sonicator ») ou, pour des préparations de grand volume, dans un appareil à émulsion de dimension industrielle. En général, le mélange à deux phases est soumis aux ultrasons pendant environ 3 à 5 minutes ou jusqu'à ce que soit un mélange clair à une phase soit une émulsion homogène se forme. Ceci est obtenu en plaçant simplement le réacteur dans un bain permettant l'application d'ondes ultrasonores à un niveau optimal. L'émulsification peut être réalisée à des températures comprises dans un large domaine, c'est-à-dire entre environ 10 °C et environ 50 °C, avantageusement à une température comprise entre 0 et 20 °C. Les conditions optimales sous lesquelles l'émulsification est réalisée dépendent du solvant, du phospholipide et du volume de la phase aqueuse utilisés dans la préparation. On peut procéder de façon empirique pour déterminer les conditions optimales pour l'émulsification. L'émulsion est alors traitée pour extraire une partie substantielle du solvant organique inerte. Ceci peut être réalisé avantageusement en utilisant un évaporateur rotatif, à une température d'environ 20 °C à 60 °C et sous pression réduite, c'est-à-dire sous vide : 1 333 à 6 665 Pa (10 à 50 mm Hg). La température mise en œuvre pour l'évaporation du solvant organique à partir de l'émulsion dépend du point d'ébullition du solvant organique particulier utilisé dans l'émulsion et de la stabilité du matériau biologiquement actif devant être encapsulé. Pendant l'évaporation, l'émulsion prend d'abord la forme d'un gel visqueux, qui constitue un produit intermédiaire. Le gel est stable et peut être conservé dans cet état pendant de courtes périodes de temps, jusqu'à une semaine (au moins) à 4 °C sous une atmosphère inerte telle que sous azote. Une petite quantité d'eau ou de solution tampon peut alors être ajoutée au gel et le mélange résultant évaporé pendant une période supplémentaire (environ 15 minutes) pour favoriser l'extraction de traces résiduelles du solvant organique et pour accélérer la transformation du gel en une suspension d'apparence homogène de capsules lipidiques oligolamellaires. Le gel peut être transformé par agitation ou par dispersion dans un milieu aqueux tel qu'une solution tampon. Les capsules obtenues présentent un diamètre compris entre environ 0,2000 et 0,4 μm (en moyenne). Une proportion substantielle des produits pharmaceutiques, chimiques, des macromolécules ou d'autres composés et des matériaux biologiques à encapsuler contenus dans la solution tampon aqueuse est encapsulée à l'intérieur des capsules lipidiques (jusqu'à environ 60 %, en fonction de la quantité de lipides, du volume de la phase aqueuse, du rapport phase organique/phase aqueuse/lipides, du type de solvant(s) organique(s) inerte(s) et du type de lipide(s) utilisés dans le procédé). Le matériau aqueux non incorporé peut être extrait si nécessaire par des techniques appropriées et connues, par exemple par centrifugations répétées, chromatographie sur colonne, chromatographie d'échange d'ions, dialyse et procédures analogues. Les capsules lipidiques avec leur contenu encapsulé peuvent alors être mises en suspension dans tout tampon isotonique pour l'utilisation. Les capsules peuvent être stérilisées en les faisant passer à travers un filtre 0,4 μm (nucléopore) quand une stérilisation est souhaitée.

Comme exemples de matériaux et composés qui peuvent être encapsulés par le procédé selon l'invention, on peut citer, mais de façon non limitative, des produits chimiques tels que la citosine arabinoside, et ses dérivés phosphorylés ; des produits chimiques tels que le 3′,5′-adénosine monophosphate cyclique, le sucrose, des antibiotiques tels que la pénicilline et la streptomycine, des hormones polypeptidiques telles que l'insuline, l'oxytocine et la vasopressine, des macromolécules telles que le dextrane, des protéines telles que l'albumine, la ferritine et des immunoglobulines G ; des enzymes tels que la phosphatase alcaline ; des acides nucléiques tels que l'acide polyadénylique (poly A), des acides ribonucléiques, des acides désoxyribonucléiques, des virus et des bactéries tels que *C. parvum* et des matériaux analogues.

De façon avantageuse, le procédé selon l'invention est mis en œuvre sous atmosphère inerte. Par le terme « atmosphère inerte » tel qu'utilisé ici, on entend une atmosphère non oxydante telle qu'une atmosphère d'azote, d'argon ou de gaz inerte analogue.

De la description ci-dessus, on observera que le procédé selon l'invention se différencie des procédés de l'art antérieur dans différentes voies. Par exemple, conformément au procédé selon l'invention, le matériau destiné à être encapsulé est ajouté à la phase organique contenant le lipide dans lequel il doit être totalement encapsulé. En outre, la phase organique est extraite en grande partie avant qu'un excès de phase aqueuse soit ajouté. L'émulsification de la phase aqueuse initiale dans la phase organique et l'extraction de la phase organique avant l'addition de tout excès de phase aqueuse sont essentielles pour un pourcentage d'encapsulage ou d'incorporation élevé dans ce procédé et constituent une différence critique entre le procédé selon l'invention et tous les procédés antérieurs décrits ci-dessus. Par le procédé selon l'invention, on obtient des capsules oligolamellaires de grande dimension à partir de lipides différents utilisés soit seuls soit en combinaisons. Un avantage du procédé de l'invention réside dans le fait que l'évaporation de la phase organique est réalisée sous des conditions de température douces et sous vide, pour éviter le risque d'inactivation de molécules sensibles.

Dans les exemples suivants, on décrit à titre non limitatif le procédé de fabrication selon l'invention et diverses applications. Dans tous les procédés décrits ci-dessous, on peut inclure de 0,5 à 1 mole d'un analogue phospholipidique fluorescent tel que le NBD-PE (ou N-4-nitrobenzo-2-oxa-1,3-diazole-phosyhatidyléthanolamine) (Avanti Biochemicals) au composé lipidique ou phospholipidique afin de pouvoir suivre visuellement la séparation des capsules ou vésicules sur une colonne.

## 0 004 223

Exemple 1 : Encapsulage d'enzymes.

Sur un ballon à fond rond de 50 ml à long col, on monte un raccord 24/40 de façon à pouvoir le raccorder à un évaporateur à vaporisation instantanée. Le flacon est également équipé de raccords pour le purger en continu avec de l'azote gazeux. On remplit le flacon de 10 micromoles de phosphatidylglycérol, 40 micromoles de phosphatidylcholine et 50 micromoles de cholestérol dissoutes dans du chloroforme. Par évaporation rotative, le solvant est évaporé, ce qui laisse une couche lipidique sur les parois internes du flacon. Le flacon est alors purgé à l'azote gazeux et 5 ml de diéthyl éther sont ajoutés sous agitation pour dissoudre les lipides. On ajoute ensuite dans le flacon 1,5 ml d'un mélange aqueux de 10 mM de chlorure de sodium tamponné à un pH de 7,4 avec 4 mM d'histidine/acide 2-{ [tris-(hydroxyméthyl) méthyl] amino } éthanesulfonique (appelé ci-après « TES ») et 10 mg/ml de phosphatase alcaline ; on obtient un mélange hétérogène à deux phases. Le mélange est alors émulsifié sous ultrasons pendant 5 minutes à 0 °C dans un dispositif ultrasonore du type à bain (modèle T-80-80-IRS, Laboratory Supplies, Hicksville, New York). L'émulsion obtenue est alors évaporée sur un évaporateur rotatif (Buchi) à une température de 25 °C et sous pression réduite (environ 1 333 à 6 665 Pa) en utilisant une pompe à eau jusqu'à ce qu'on obtienne un gel visqueux. Ce gel est un précurseur intermédiaire des liposomes ou capsules synthétiques qu'on veut préparer. Le gel est stable et il peut être conservé pendant des périodes d'au moins une semaine à une température d'environ 4 °C sous atmosphère inerte.

Au gel obtenu ci-dessus, on ajoute 1,5 ml de la solution tampon chlorure de sodium/histidine/TES décrite ci-dessus et on fait tourner doucement le flacon pour obtenir une suspension aqueuse du gel. Le mélange résultant est évaporé à 30 °C sous une pression d'environ 1 333 à 6 665 Pa pendant 15 autres minutes et on obtient une suspension opaque de capsules de phospholides (liposomes synthétiques) présentant un diamètre moyen de 0,2 à 0,6 $\mu$m. Par une évaporation prolongée sans addition supplémentaire de solution tampon, on obtient des suspensions similaires. L'examen au microscope électronique montre que les capsules sont sensiblement des capsules oligalomellaires.

On fait passer la suspension opaque de capsules dans une colonne remplie de bio-gel A 1,5 agarose pour séparer les capsules oligolamellaires du mélange de phosphatase alcaline non encapsulé. Le pourcentage d'encapsulage s'avère être de 34 %. Les capsules séparées sont mises en suspension dans toute solution tampon isotonique et utilisées en tant que matériau de départ pour une réaction enzymatique.

De façon similaire, en répétant la procédure décrite ci-dessus pour l'exemple 1 mais en remplaçant la phosphatase alcaline telle qu'utilisée par 10 mg/ml de L-asparaginase, on obtient des capsules synthétiques dans lesquelles la L-asparaginase est encapsulée. Les capsules sont utiles en tant qu'elles inhibent la croissance de certaines tumeurs chez les mammifères ; voir Chang, T., Nature *229*, 117-118 (1971) pour la technique d'utilisation.

De façon similaire, en répétant la procédure ci-dessus de l'exemple 1, mais en remplaçant la phosphatase alcaline par 10 mg/ml de divers glycosidases, on obtient des capsules synthétiques dans lesquelles les enzymes sont encapsulés. Les capsules sont utiles dans une thérapie par remplacement d'enzymes ; voir « Enzyme Therapy in Lycosomal Storage Diseases », Tager, Hooghwinkel and Daems ; North-Hullang Publ. Co., (1974) pour la technique d'utilisation.

De façon similaire, en répérant le processus de l'exemple 1, mais en remplaçant la phosphatase alcaline par 84 mg/ml de 1-β-D-arabinofuranosylcytosine (appelé ci-après « ara-C »), on obtient des capsules synthétiques dans lesquelles l'arabinofuranosylcytosine est encapsulée, capsules qui peuvent être utilisées pour inhiber la croissance de certaines tumeurs chez les mammifères ; voir Mayhew et al., Cancer Research, *36* : 4 406-4 411, Décembre 1976, pour la technique d'utilisation. De façon analogue, des analogues nucléosides d'ara-C, du méthotrexate ou des anti-métabolites analogues peuvent être encapsulés pour être utilisés dans le but d'inhiber la croissance de certaines tumeurs chez les mammifères.

En répétant la procédure de l'exemple 1 décrite ci-dessus mais en remplaçant la phosphatase alcaline par 0,6 mg/ml d'actinomycine D, ce composé est encapsulé. L'actinomycine D ainsi encapsulée peut être administrée à des patients atteints de cancers, suivant la méthode de Gregoriadis et al., Lancet, 29 juin 1974, pages 1 313-1 316 ; voir également D. Papahadjopoulos et al., Cancer Research, *36* : 2 988-2 994, Septembre 1976.

En répétant de façon similaire le processus de l'exemple 1, mais en remplaçant la phosphatase par le sel de sodium de l'héparine dissous dans une solution saline tamponnée au phosphate, on obtient des capsules synthétiques dans lesquelles l'héparine est encapsulée. Ces capsules peuvent être mises en suspension dans une solution tampon et administrées par voie intraveineuse à un mammifère en tant qu'anticoagulant à délivrance lente ou prolongée, pour traiter des affections répondant à un tel traitement.

Des médicaments analogues tels que le méglumine antimoniate peuvent être encapsulés en suivant le même processus que celui de l'exemple 1. Le méglumine antimoniate encapsulé par ce processus peut être utilisé chez les mammifères contre des organismes parasites tels que les *Leishmaniasis.*

En répétant de façon similaire le processus de l'exemple 1, mais en remplaçant la phosphatase par des composés chélateurs de métaux tels que l'acide éthylènediaminetétraacétique (EDTA), la pénicilline, et analogues, on obtient des capsules synthétiques qui peuvent être utilisées pour le traitement de patients souffrant d'empoisonnements par des métaux, de problèmes de rétention de métaux ou de certaines anémies ; voir Brevet Etats-Unis n° 4 016 290.

Exemple 2 : Encapsulage d'acides nucléiques.

On introduit dans le flacon à fond rond décrit dans l'exemple 1 ci-dessus 10 micromoles de phosphatidylglycérol, 40 micromoles de phosphatidylcholine et 50 micromoles de cholestérol dissoutes dans du chloroforme. Le milieu réactionnel est soumis à une évaporation sur un évaporateur rotatif et on obtient un dépôt d'une mince couche lipidique sur les parois internes du flacon. Le ballon est purgé à l'azote gazeux et on ajoute 5 ml de diéthyl éther dans le flacon sous agitation pour redissoudre les lipides. On ajoute ensuite 1,5 ml d'un mélange aqueux de 10 mM de chlorure de sodium tamponné à un pH de 7,4 avec 4 mM d'histidine/TES et 1 mg/ml d'acide ribonucléique (RNA) (soit l'acide polyadénylique, soit le 25 S tétrahyména robosomal RNA).

Le mélange résultant est émulsifié par ultrasons pendant 5 mn à une température de 0 °C dans le dispositif à ultrasons du type à bain (Laboratory Supplies, voir ci-dessus). L'émulsion obtenue est ensuite soumise à une évaporation à une température de 0 °C et sous une pression de 1 333 à 6 665 Pa (10-50 mm Hg) sur l'évaporateur à évaporation instantanée jusqu'à ce qu'un gel se forme. On ajoute au gel 1,5 ml de la solution tampon chlorure de sodium/histidine/TES décrite ci-dessus et l'évaporation est poursuivie pendant 15 autres minutes. On obtient une suspension opaque de capsules synthétiques dans lesquelles l'acide ribonucléique est encapsulé. Après maintien à une température de 20 °C pendant environ 30 mn, la suspension est soumise à une centrifugation à 100 000 G pendant 30 mn. Le surnageant est alors extrait, les capsules synthétiques étant obtenues en tant que sédiment. Le pourcentage d'encapsulage de RNA s'avère être de 40 à 43 %. Les capsules sont utiles pour introduire le RNA à travers des membranes cellulaires.

De façon similaire, d'autres polyribonucléotides tels que l'acide polyinosinique-polycytosinique, ou d'autres polynucléotides synthétiques, peuvent être substitués à l'acide ribonucléique utilisé dans le processus de l'exemple 2, pour former des capsules lipidiques synthétiques dans lesquelles ces composés sont encapsulés et peuvent être utilisés en tant qu'agents antiviraux. Les techniques d'utilisation de telles capsules sont bien connues.

Exemple 3 : Encapsulage d'insuline.

On introduit dans le ballon à fond rond décrit dans l'exemple 1 ci-dessus 50 micromoles de dipalmitoyl phosphatidylcholine et 50 micromoles de cholestérol dans du chloroforme. Le mélange réactionnel est soumis à une évaporation sur un évaporateur rotatif et le mélange de lipides se dépose sur les parois internes du ballon. Le ballon est alors purgé à l'azote gazeux et on ajoute 7,5 ml d'éther isopropylique et 7,5 ml de chloroforme sous agitation pour redissoudre les lipides. On ajoute à la solution 1,5 ml d'un mélange aqueux d'insuline (d'origine bovine, 25 mg/ml dans 7 M urée) dans une solution tampon aqueuse (10 mM de chlorhydrate de triéthylamine ; pH 7,9). Le mélange résultant est alors émulsifié par ultrasons pendant 5 mn à une température de 45 °C dans un appareil à ultrasons à bain (Laboratory Supplies, voir ci-dessus). L'émulsion est ensuite soumise à une évaporation à une température de 45 °C et sous une pression de 1 333 à 6 665 Pa sur un évaporateur à vaporisation instantanée jusqu'à ce qu'un gel se forme. On ajoute au gel 1,5 ml de la solution tampon chlorure de sodium/TES décrite précédemment, sous agitation. On poursuit alors l'évaporation pendant 15 autres minutes. On laisse reposer la suspension opaque résultante pendant 30 mn à une température de 45 °C. On fait alors passer la suspension à travers une colonne G-75 Sephadex à une température ambiante (environ 26 °C) pour séparer les capsules lipidiques synthétiques dans lesquelles la solution d'insuline est encapsulée. Le pourcentage d'insuline encapsulée est calculé comme étant égal à 34 %. Les capsules peuvent être mises en suspension dans une solution saline tamponnée au phosphate et administrées par voie orale ou intramusculaire suivant des doses usuelles à des patients souffrant de diabète contrôlé par insuline. Le processus ci-dessus peut être répété à des températures inférieures pendant le traitement aux ultrasons et l'évaporation (0-20 °C) si on utilise des phospholipides fluides pour la formation des capsules à la place de dipalmitoylphosphatidylcholine.

De façon similaire, en répétant le processus ci-dessus mais en remplaçant l'insuline par d'autres hormones peptidiques solubles dans l'eau, telles que la vasoprésine, la somatostatine ou leurs dérivés synthétiques et analogues, on obtient des capsules lipidiques synthétiques dans lesquelles ces matériaux sont encapsulés et qui peuvent être utilisées pour le traitement thérapeutique de mammifères atteints de maladies répondant à de tels traitements.

Comme mentionné ci-dessus, la force ionique de la solution aqueuse destinée à être encapsulée constitue un facteur déterminant pour le degré d'encapsulage obtenu dans le procédé selon l'invention. Quand la force ionique de la solution aqueuse destinée à être encapsulée augmente, à la fois le pourcentage d'encapsulage et le volume de la phase aqueuse encapsulée par micromole de phospholipide diminuent. Des concentrations élevées en sucrose, glycérol, urée et analogues n'ont pas le même effet d'augmentation de la concentration en ions dans le mélange destiné à être encapsulé. Cet effet est montré dans l'exemple 4.

### Exemple 4

On répète six fois le processus de l'exemple 1 ci-dessus mais, dans chaque cas, la phosphatase alcaline utilisée dans l'exemple 1 est remplacée par 0,84 mg/ml de 1-β-D-arabinofuranosylcytosine (ara-C) et la proportion de chlorure de sodium est modifiée dans chaque cas pour modifier la force ionique du mélange ara-C destiné à être utilisé. La proportion de chlorure de sodium présent et le degré résultant d'encapsulage obtenu sont montrés dans le Tableau 1 ci-dessous.

TABLEAU 1

| Essai n° | Moles NaCl | Pourcentage d'encapsulage |
|---|---|---|
| 1 | 0,50 | 15,0 |
| 2 | 0,15 | 37,5 |
| 3 | 0,10 | 42,5 |
| 4 | 0,04 | 47,5 |
| 5 | 0,02 | 62,3 |
| 6 | 0,00 | 62,5 |

La concentration en lipides dans le mélange à deux phases soumis à l'émulsification agit également sur le pourcentage d'encapsulage obtenu par le procédé selon l'invention. Par exemple, le pourcentage d'encapsulage d'ara-C diminue quand les concentrations totales en lipides diminuent. Cependant, le volume aqueux encapsulé par mole de phospholipide augmente.

Un volume aqueux d'environ 11,2 litres par mole de phospholipide est encapsulé quand la teneur totale en lipides est d'environ 100 micromoles par 5 ml de solvant et augmente jusqu'à environ 22,5 litres par mole de phospholipide quand la teneur totale en lipides est réduite à 20 micromoles. Le domaine de 20-100 µM/5 ml est préféré et, au-dessus de 100 µM, on obtient un plus grand nombre de capsules multilamellaires. L'exemple 5 ci-dessous illustre le pourcentage d'encapsulage d'ara-C obtenu pour différentes concentrations en lipides.

### Exemple 5

On répète cinq fois le processus de l'exemple 1 ci-dessus, mais dans chaque cas on remplace la phosphatase alcaline par 84 mg/ml de 1-β-D-arabinofuranosylcytosine (ara-C) et la concentration totale en lipides est différente dans chaque essai (le même rapport PG/PC/cholestérol étant maintenu). La proportion totale de lipides utilisée dans chaque essai et le pourcentage d'encapsulage obtenu sont montrés dans le Tableau 2 ci-dessous.

TABLEAU 2

| Essai n° | Teneur totale en lipides (µ moles) | Pourcentage d'encapsulage |
|---|---|---|
| 1 | 20 | 15 |
| 2 | 40 | 25 |
| 3 | 60 | 30 |
| 4 | 80 | 31,2 |
| 5 | 100 | 37,5 |

La nature du lipide ou des lipides utilisés dans le procédé selon l'invention n'apparaît pas être critique. A l'exception des lipides chargés négativement quand utilisés seuls, tels que PS, PG, cardiolipine ou acide phosphatidique, différentes compositions de lipides dans des proportions similaires lipides/solvant/tampon ne présentent qu'un effet modéré sur le degré d'encapsulage obtenu. L'exemple 6 ci-dessous illustre ce fait.

### Exemple 6

On répète la procédure de l'exemple 1 ci-dessus sept fois, à l'exception du fait qu'on utilise 0,4 mg/ml d'ara-C au lieu de la phosphatase alcaline ou 0,01 M de chlorure de sodium dans une solution saline Dulbecco tamponnée au phosphate 1/10 à la place de TES et, dans chaque essai, le composé lipidique est différent. Dans l'essai n° 4, on utilise 3,5 ml de diéthyl éther et 1,05 ml de la solution saline Dulbecco (PBS) ; dans l'essai n° 5, on utilise 7,5 ml d'éther isopropylique avec 7,5 ml de chloroforme à la place de l'éther diéthylique et la seconde évaporation est réalisée à 45 °C ; dans l'essai n° 6, on utilise un mélange de 7,5 ml d'éther isopropylique et 7,5 ml d'éthanol à la place de l'éther diéthylique ; dans les essais n° 7 et 8, on utilise un mélange de 5 ml d'éther diéthylique avec 1 ml de méthanol à la place de l'éther diéthylique seul. Les lipides utilisés, la concentration en lipides utilisée et le pourcentage d'encapsulage obtenu dans chaque essai sont montrés dans le Tableau 3 ci-dessous.

TABLEAU 3

| Essai n° | Composition de lipides | Teneur totale en lipides ($\mu$ moles) | Pourcentage d'encapsulage ara-C | Pourcentage d'encapsulage sodium |
|---|---|---|---|---|
| 1 | PG/PC/Chol* (1/4/5) | 100 | 62 | 47,5 |
| 2 | PG/PC (1/4) | 50 | — | 34,3 |
| 3 | PS/PC/Chol (1/4/5) | 100 | 61 | — |
| 4 | Stam**/PC/Chol (1/4/3) | 56 | — | 44,6 |
| 5 | DPPC | 50 | — | 40,6 |
| 6 | Sphingomyéline | 66 | 24 | — |
| 7 | PG | 50 | — | 18,6 |
| 8 | PS | 50 | — | 22 |

\* Chol représente cholestérol.
\*\* Stam représente stéarylamine.

Note :

Solution saline Dulbecco tamponnée au phosphate (NaCl ; 0,137 M ;
NaHPO$_4$, 8,2 × 10$^{-3}$ M ;
KCl 2,7 × 10$^{-3}$ M ; KH$_2$PO$_4$ 1,9 × 10$^{-3}$ M ; MgCl$_2$ 1,1 × 10$^{-3}$ M ; CaCl$_2$ 0,9 × 10$^{-3}$ M).

Les capsules préparées par le procédé selon l'invention ont un rôle de porteur pour le matériau biologiquement actif encapsulé qui reste bio-disponible, c'est-à-dire qui conserve ses propriétés biologiques. Les parois des capsules sont imperméables aux matériaux encapsulés, comme montré dans l'exemple 7.

Exemple 7

Trois portions de 10 ml des capsules préparées selon l'essai n° 5 de l'exemple 4 sont soumises à une séparation et à une dialyse pendant une nuit pour extraire le matériau non-encapsulé présent dans la suspension de capsules renfermant l'ara-C. Chaque portion dialysée est alors placée dans un sac de dialyse et dialysée à l'encontre de trois portions successives de 10 ml de solution saline Dulbecco tamponnée au phosphate, chaque fois pendant une heure à diverses températures. Le pourcentage d'ara-C ayant diffusé par heure à travers le sac de dialyse est alors observé. Les observations, températures utilisées et durées de dialyse sont montrées dans le Tableau 4 ci-dessous.

TABLEAU 4
Pourcentage du matériau encapsulé libéré par heure

| Durée | Température en °C 10 | 20 | 37 |
|---|---|---|---|
| 1 heure | 0,376 | 0,697 | 1,97 |
| 2 heures | 0,221 | 0,613 | 1,69 |
| 3 heures | 0,195 | 0,649 | 1,54 |

Exemple 8 : Encapsulage de bactéries.

On introduit dans le ballon à fond rond décrit dans l'exemple 1 ci-dessus, 50 micromoles de PG/PC (1/4) et 50 moles de cholestérol dissoutes dans du chloroforme. Par évaporation rotative, le solvant est évaporé, ce qui laisse un film du mélange de lipides sur les parois intérieures du ballon. On purge alors le ballon avec de l'azote gazeux et on introduit 5 ml de diéthyl éther sous agitation pour redissoudre les lipides. On ajoute à la solution 1,5 ml d'une solution aqueuse de bactéries *C. parvum* tuées par action de la chaleur dans une solution saline de Dulbecco tamponnée au phosphate. Le mélange résultant est soumis à une émulsification par ultrasons pendant 5 mn à 0 °C dans un appareil à ultrasons (Laboratory Supplies, voir ci-dessus). L'émulsion est alors soumise à évaporation à une température de 20 °C et sous une pression réduite de 1 333 à 6 665 Pa dans un évaporateur à vaporisation instantanée jusqu'à ce qu'un gel se forme. On ajoute au gel 1,5 ml de la solution saline Dulbecco tamponnée au phosphate et l'évaporation est poursuivie pendant 15 autres minutes. Les *C. parvum* encapsulés sont séparés des *C.*

0 004 223

*parvum* non-encapsulés par centrifugation sur un support sucrose. 30 % des *C. parvum* sont encapsulés. Les capsules ainsi obtenues peuvent être utilisées en tant qu'adjuvants pour une immunothérapie contre certains cancers, comme décrit en détail dans Seminars Oncol. *1* : 367-378, 1974.

En répétant de façon similaire la procédure décrite ci-dessus pour l'exemple 8, mais en remplaçant le *C. parvum* par d'autres immunostimulants tels que le BCG, des cellules cancéreuses intactes ou des fractions purifiées de ces dernières, on obtient des capsules synthétiques renfermant ces matériaux. Ces capsules pourraient être utiles dans le cadre d'immunothérapies quand administrées à des mammifères souffrant d'affections répondant à de tels traitements.

L'exemple 8 montre également un avantage particulier du procédé selon l'invention en ceci qu'il fournit un moyen non seulement pour encapsuler des matériaux en solution, mais également aussi bien des matériaux en suspension dans un milieu aqueux. Le matériau destiné à être encapsulé peut être dissous dans la phase aqueuse, s'il est soluble. En variante, le matériau peut être finement divisé et mis en suspension dans le milieu aqueux ou ce peuvent être des macromolécules en suspension, comme dans le cas de virus ou de matériaux cellulaires. Il apparaît que si la capsule présente l'espace nécessaire pour le matériau en suspension, celui-ci peut être encapsulé par le procédé selon l'invention. Ceci constitue un avantage du procédé de l'invention étant donné que, par de nombreux procédés d'encapsulage selon l'art antérieur, il était possible d'encapsuler des matériaux autrement que dissous.

Les capsules préparées par le procédé selon l'invention se sont avérées comme pouvant renfermer de grands volumes et des matériaux de dimension relativement grande ayant une activité biologique. Suivant la nature des matériaux encapsulés, les capsules peuvent être utilisées dans un large domaine d'applications. Par exemple, quand des antibiotiques sont encapsulés, elles peuvent être utilisées in vivo et in vitro pour traiter les germes pathogènes résistant aux antibiotiques comprenant les bactéries gram-négatif *E. coli, H. influenzae, P. aeruginosa* et analogues. Une théorie a été exprimée selon laquelle la résistance aux médicaments que montrent les organismes bactériens résulte d'un développement de l'imperméabilité de la paroi cellulaire. L'administration d'un antibiotique approprié à l'organisme est facilitée quand elle est réalisée par l'intermédiaire de capsules lipidiques, étant donné que la capsule lipidique semble pénétrer facilement la paroi cellulaire des bactéries, libérant l'antibiotique encapsulé à l'intérieur du germe pathogène.

De façon similaire, comme décrit rapidement ci-dessus, par encapsulage de composés antiparasites, on peut administrer le matériau encapsulé au système réticuloendothélial d'un mammifère. La capsule lipidique peut libérer de façon efficace les médicaments pour neutraliser le parasite avec un effet toxique minimal sur le mammifère.

L'encapsulage d'agents viraux et bactériens tels que le virus de la polio inactivé ou le *P. aeruginosa* fournit un vaccin qui peut être administré à un mammifère pour conférer à ce mammifère une immunité à l'égard des maladies provoquées par l'agent, avec une réduction du risque de toxicité associé aux vaccins qui ne sont pas encapsulés conformément au procédé selon l'invention.


**Revendications**

1. Procédé de fabrication de capsules synthétiques oligolamellaires lipidiques propres à renfermer un (ou des) matériau(x) biologiquement actif(s), caractérisé en ce que :
on prépare un mélange d'un composé formateur de parois des capsules dans un solvant organique et d'un mélange aqueux du matériau biologiquement actif destiné à être encapsulé, le rapport de la phase organique à la phase aqueuse étant d'une valeur propre à fournir une émulsion du type eau-dans-huile,
on forme une émulsion homogène du type eau-dans-huile dudit mélange,
on évapore le solvant organique de l'émulsion jusqu'à ce qu'on obtienne un mélange ayant le caractère d'un gel, et
on transforme le mélange ayant le caractère d'un gel en une suspension de capsules oligolamellaires synthétiques renfermant le matériau biologiquement actif soit en agitant ledit mélange ayant le caractère d'un gel, soit en dispersant ledit mélange ayant le caractère d'un gel dans un milieu aqueux.

2. Procédé selon la revendication 1, caractérisé en ce que le composé formateur de parois est un phospholipide.

3. Procédé selon la revendication 2, caractérisé en ce que le phospholipide est de la phosphatidylcholine mélangée à un second phospholipide et à du cholestérol.

4. Procédé selon la revendication 3, caractérisé en ce que la phosphatidylcholine est dans un rapport 4/l par rapport au second phospholipide.

5. Procédé selon la revendication 1, caractérisé en ce que le composé formateur de parois est fourni au départ dans un solvant inerte et est ensuite déposé sur la paroi d'un réacteur par évaporation du solvant inerte.

6. Procédé selon la revendication 1, caractérisé en ce que le solvant organique est du diéthyléther, du chloroforme, du tétrahydrofuranne ou de l'éther isopropylique.

7. Procédé selon la revendication 1, caractérisé en ce que le mélange aqueux est tamponné à un pH approprié pour maintenir à l'état stable le matériau biologiquement actif.

10

8. Procédé selon la revendication 7, caractérisé en ce que le tampon est un composé ionique présentant une force ionique inférieure à 0,3.

9. Procédé selon la revendication 1, caractérisé en ce que le composé formateur de parois est utilisé à raison d'environ 0,5 mg à environ 50 mg/ml de solvant organique.

10. Procédé selon la revendication 1, caractérisé en ce que l'émulsification est réalisée par exposition à des ondes ultrasonores à une température comprise entre environ −10 °C et environ 50 °C.

11. Procédé selon la revendication 1, caractérisé en ce que l'eau ajoutée au mélange analogue à un gel est tamponnée à un pH approprié pour maintenir à l'état stable le matériau biologiquement actif.

12. Procédé selon la revendication 1, caractérisé en ce qu'il est mis en œuvre sous une atmosphère inerte.

13. Procédé selon la revendication 1, caractérisé en ce que la transformation a lieu par dispersion dans de l'eau tamponnée à un pH approprié pour maintenir à l'état stable le matériau biologiquement actif.

14. Procédé selon la revendication 1, caractérisé en ce que les capsules oligolamellaires sont ensuite séparées du ou des matériau(x) biologiquement actif(s) qui n'ont pas été encapsulés.

15. Procédé selon la revendication 1, caractérisé en ce que le matériau biologiquement actif est un enzyme.

16. Procédé selon la revendication 1, caractérisé en ce que le matériau biologiquement actif est un antibiotique.

17. Procédé selon la revendication 1, caractérisé en ce que le matériau biologiquement actif est un acide nucléique.

18. Procédé selon la revendication 17, caractérisé en ce que l'acide nucléique est un acide désoxyribonucléique.

19. Procédé selon la revendication 1, caractérisé en ce que le matériau biologiquement actif est un pesticide.

20. Procédé selon la revendication 1, caractérisé en ce que le matériau biologiquement actif est un polypeptide.

21. Procédé selon la revendication 20, caractérisé en ce que le polypeptide est de l'insuline.

22. Procédé selon la revendication 1, caractérisé en ce que le matériau biologiquement actif est un organisme bactérien.

23. Procédé selon la revendication 1, caractérisé en ce que le matériau biologiquement actif est un virus.

24. Procédé selon la revendication 1, caractérisé en ce que le composé formateur de parois est un mélange de cholestérol, phosphatidylglycérol et phosphatidylcholine dans un rapport 5/1/4 en poids.

25. Produit obtenu par la mise en œuvre du procédé selon l'une quelconque des revendications 1 à 24.

26. Composition pesticide comprenant un mélange aqueux de pesticide encapsulé dans une capsule lipidique oligolamellaire.

27. Procédé de destruction d'insectes, caractérisé en ce qu'on applique à l'emplacement où ils se trouvent une quantité efficace d'un pesticide efficace encapsulé dans une capsule lipidique oligolamellaire.

28. Procédé de fertilisation agricole, caractérisé en ce qu'on applique aux cultures une quantité nutritive d'un agent de nutrition pour les cultures encapsulé dans une capsule lipidique oligolamellaire.

29. Procédé pour introduire un acide désoxyribonucléique ou des fragments d'un tel acide dans une cellule vivante, caractérisé en ce qu'on encapsule l'acide ou le fragment de l'acide dans une capsule lipidique et en ce qu'on met la capsule en contact avec ladite cellule, l'introduction se produisant alors spontanément.

## Claims

1. A process for producing synthetic oligolamellar lipid capsules suitable for enclosing one (or several) biologically active material(s), characterised by preparing a mixture of a composition forming the walls of the capsules in an organic solvent and of an aqueous mixture of the biologically active material to be encapsulated, the proportion of the organic phase to the aqueous phase having a value suitable to provide a water-in-oil type of emulsion, forming a homogeneous water-in-oil type of emulsion from said mixture, evaporating the organic solvent from the emulsion until a mixture of gel character is obtained, and converting the mixture of gel character into a suspension of synthetic oligolamellar capsules enclosing the biologically active material, by either agitating said mixture of gel character or dispersing the mixture of gel character in an aqueous environment.

2. A process according to claim 1, characterised in that the composition forming the walls is a phospholipid.

3. A process according to claim 2, characterised in that the phospholipid is phosphatidyl choline mixed with a second phospholipid and cholesterol.

4. A process according to claim 3, characterised in that the phosphatidyl choline is present in a proportion of 4/1 relative to the second phospholipid.

5. A process according to claim 1, characterised in that the composition forming the walls is initially supplied in an inert solvent and is subsequently deposited on the walls of a reactor by evaporation of the inert solvent.

6. A process according to claim 1, characterised in that the organic solvent is diethyl ether, chloroform, tetrahydrofurane or isopropyl ether.

7. A process according to claim 1, characterised in that the aqueous mixture is buffered at a pH suitable for maintaining the biologically active material in a stable condition.

8. A process according to claim 7, characterised in that the buffer is an ionic composition having an ionic strength of less than 0.3.

9. A process according to claim 1, characterised in that the compound forming the walls is used in a ratio of about 0.5 mg to about 50 mg/ml relative to the organic solvent.

10. A process according to claim 1, characterised in that the emulsification is achieved by exposure to ultrasonic waves at a temperature between about −10 °C and about 50 °C.

11. A process according to claim 1, characterised in that the water added to the gel-like mixture is buffered at a pH suitable for maintaining the active biological material in a stable condition.

12. A process according to claim 1, characterised in that it is carried out in an inert atmosphere.

13. A process according to claim 1, characterised in that the conversion takes place by dispersion in water buffered at a pH suitable for maintaining the biologically active material in a stable condition.

14. A process according to claim 1, characterised in that the oligolamellar capsules are subsequently separated from biologically active material which has not become encapsulated.

15. A process according to claim 1, characterised in that the biologically active material is an enzyme.

16. A process according to claim 1, characterised in that the biologically active material is an antibiotic.

17. A process according to claim 1, characterised in that the biologically active material is a nucleic acid.

18. A process according to claim 17, characterised in that the nucleic acid is a deoxyribonucleic acid.

19. A process according to claim 1, characterised in that the biologically active material is a pesticide.

20. A process according to claim 1, characterised in that the biologically active material is a polypeptide.

21. A process according to claim 20, characterised in that the polypeptide is insulin.

22. A process according to claim 1, characterised in that the biologically active material is a bacterial organism.

23. A process according to claim 1, characterised in that the biologically active material is a virus.

24. A process according to claim 1, characterised in that the composition forming the walls is a mixture of cholesterol, phosphatidyl glycerol and phosphatidyl choline in a ratio of 5/1/4 by weight.

25. The product obtained by carrying out the process according to any one of claims 1 to 24.

26. A pesticide composition consisting of an aqueous pesticide mixture encapsulated in an oligolamellar lipid capsule.

27. A process for destroying insects, characterised by applying at the site on which they are located an effective amount of an effective pesticide encapsulated in an oligolamellar lipid capsule.

28. A process of agricultural fertilisation, characterised by applying to the cultures a nutrious amount of a nutritive for the cultures which is encapsulated in an oligolamellar lipid capsule.

29. A process for introducing a deoxyribonucleic acid or fragments of an acid of this kind into a living cell, characterised by encapsulating the acid or the fragment of the acid in a lipid capsule and placing the capsule in contact with said cell, the introduction then occurring spontaneously.

## Ansprüche

1. Verfahren zur Herstellung von synthetischen oligolamellaren Lipidkapseln, die zum Einschließen eines oder mehrerer biologisch aktiver Materialien geeignet sind, dadurch gekennzeichnet, daß man eine Mischung aus einer die Wände der Kapseln bildenden Zusammensetzung in einem organischen Lösungsmittel und einer wässrigen Mischung des einzukapselnden biologisch aktiven Materials ansetzt, in der das Mengenverhältnis der organischen Phase zur wässrigen Phase einen Wert aufweist, bei dem sich eine Emulsion von der Art Wasser-in-Öl ergibt, daß man aus der Mischung eine homogene Emulsion von der Art Wasser-in-Öl bildet, daß man das organische Lösungsmittel aus der Emulsion verdampft bis eine Mischung von gelartigem Charakter erhalten wird, und daß man die Mischung von gelartigem Charakter in eine Suspension von synthetischen oligolamellaren Kapseln, die das biologisch aktive Material einschließen, entweder durch Umrühren der Mischung von gelartigem Charakter oder Dispergieren der Mischung von gelartigem Charakter in wässriger Umgebung überführt.

2. Verfahren nach Anspruch 1, dadurch gekennzeichnet, daß die die Wände der Kapseln bildende Zusammensetzung ein Phospholipoid ist.

3. Verfahren nach Anspruch 2, dadurch gekennzeichnet, daß das Phospholipoid Phosphatidylcholin vermischt mit einem zweiten Phospholipoid und Cholesterol ist.

4. Verfahren nach Anspruch 3, dadurch gekennzeichnet, daß das Phosphatidylcholin in einem Verhältnis von 4: 1 zum zweiten Phospholipoid vorhanden ist.

5. Verfahren nach Anspruch 1, dadurch gekennzeichnet, daß die die Wände bildende Zusammensetzung anfänglich in einem inerten Lösungsmittel zugeführt und danach auf den Wänden eines Reaktors durch Verdampfen des inerten Lösungsmittels abgeschieden wird.

6. Verfahren nach Anspruch 1, dadurch gekennzeichnet, daß das organische Lösungsmittel Diäthyläther, Chloroform, Tetrahydrofuran oder Isopropyläther ist.

7. Verfahren nach Anspruch 1, dadurch gekennzeichnet, daß die wässrige Mischung bei einem pH gepuffert ist, bei dem das biologisch aktive Material in beständigem Zustand gehalten wird.

8. Verfahren nach Anspruch 7, dadurch gekennzeichnet, daß der Puffer eine ionische Verbindung mit einer Ionenkonzentration von weniger als 0,3 ist.

9. Verfahren nach Anspruch 1, dadurch gekennzeichnet, daß die die Wände bildende Zusammensetzung in einem Verhältnis von ungefähr 0,5 mg bis ungefähr 50 mg/ml des organischen Lösungsmittels eingesetzt wird.

10. Verfahren nach Anspruch 1, dadurch gekennzeichnet, daß die Emulgierung durch Ultraschallwellenexponierung bei einer Temperatur zwischen ungefähr −10 °C und ungefähr 50 °C erzielt wird.

11. Verfahren nach Anspruch 1, dadurch gekennzeichnet, daß das der gelähnlichen Mischung zugegebene Wasser bei einem pH gepuffert ist, bei dem das biologisch aktive Material in beständigem Zustand gehalten wird.

12. Verfahren nach Anspruch 1, dadurch gekennzeichnet, daß es in einer inerten Atmosphäre durchgeführt wird.

13. Verfahren nach Anspruch 1, dadurch gekennzeichnet, daß das Überführen durch Dispergieren in Wasser stattfindet, welches bei einem pH gepuffert ist, bei dem das biologisch aktive Material in beständigem Zustand gehalten wird.

14. Verfahren nach Anspruch 1, dadurch gekennzeichnet, daß die oligolamellaren Kapseln danach von dem bzw. den biologisch aktiven Materialien abgetrennt werden, die nicht eingekapselt worden sind.

15. Verfahren nach Anspruch 1, dadurch gekennzeichnet, daß das biologisch aktive Material ein Enzym ist.

16. Verfahren nach Anspruch 1, dadurch gekennzeichnet, daß das biologisch aktive Material ein Antibiotikum ist.

17. Verfahren nach Anspruch 1, dadurch gekennzeichnet, daß das biologisch aktive Material eine Nukleinsäure ist.

18. Verfahren nach Anspruch 17, dadurch gekennzeichnet, daß die Nukleinsäure eine Desoxyribonukleinsäure ist.

19. Verfahren nach Anspruch 1, dadurch gekennzeichnet, daß das biologisch aktive Material ein Pestizid ist.

20. Verfahren nach Anspruch 1, dadurch gekennzeichnet, daß das biologisch aktive Material ein Polypeptid ist.

21. Verfahren nach Anspruch 20, dadurch gekennzeichnet, daß das Polypeptid Insulin ist.

22. Verfahren nach Anspruch 1, dadurch gekennzeichnet, daß das biologisch aktive Material ein Bakterienorganismus ist.

23. Verfahren nach Anspruch 1, dadurch gekennzeichnet, daß das biologisch aktive Material ein Virus ist.

24. Verfahren nach Anspruch 1, dadurch gekennzeichnet, daß die die Wände bildende Zusammensetzung eine Mischung aus Cholesterol, Phosphatidylglycerin und Phosphatidylcholin in einem Gewichtsverhältnis von 5:1:4 ist.

25. Produkt der Durchführung des Verfahrens gemäß einem der Ansprüche 1 bis 24.

26. Pestizidzusammensetzung, bestehend aus einer in einer oligolamellaren Lipidkapsel eingekapselten wässrigen Pestizidmischung.

27. Verfahren zur Vernichtung von Insekten, dadurch gekennzeichnet, daß man am Ort, an dem sie sich befinden, eine wirksame Menge eines in einer oligolamellaren Lipidkapsel eingekapselten, wirksamen Pestizids anwendet.

28. Verfahren zur landwirtschaftlichen Düngung, dadurch gekennzeichnet, daß man die Kulturen mit einer Nährmenge eines in einer oligolamellaren Lipidkapsel eingekapselten Nährstoffes behandelt.

29. Verfahren zum Einführen einer Desoxyribonukleinsäure oder Bruchstücken einer derartigen Säure in eine lebende Zelle, dadurch gekennzeichnet, daß man die Säure oder ein Bruchstück der Säure in eine Lipidkapsel einkapselt und die Kapsel mit der Zelle in Berührung bringt, wobei das Einführen dann spontan stattfindet.